Europäisches Patentamt

European Patent Office

Office européen des brevets

(11) Veröffentlichungsnummer: **0 148 312**
A2

# (12) EUROPÄISCHE PATENTANMELDUNG

(21) Anmeldenummer: **84105577.5**

(22) Anmeldetag: **16.05.84**

(51) Int. Cl.⁴: **A 61 N 1/08**

(30) Priorität: **23.12.83 DE 3346744**

(43) Veröffentlichungstag der Anmeldung: **17.07.85**
**Patentblatt 85/29**

(84) Benannte Vertragsstaaten: **DE FR GB IT NL SE**

(71) Anmelder: **MEDEL Medizinische Elektronik Handelsges. mbH, Alsterblick 55, D-2000 Hamburg 65 (DE)**

(72) Erfinder: **Timmermann, Hartwig, Alsterblick 55, D-2000 Hamburg 65 (DE)**

(74) Vertreter: **Patentanwälte Dipl.-Ing. J. Richter Dipl.-Ing. F. Werdermann, Neuer Wall 10, D-2000 Hamburg 36 (DE)**

(54) Schaltungsanordnung zum Überprüfen der Lage von Elektroden.

(57) Zum Überprüfen der Lage von Elektroden auf einen Gegenstand, der insbesondere mit von einem Generator an die Elektroden gelieferter elektrischer Energie zu behandeln ist, wird ein elektrischer Prüfimpuls periodisch an die Elektroden (6, 8) angelegt, und die bzw. der durch den Prüfimpuls an bzw. in der Übergangsimpedanz (10, 11, 12) zwischen den Elektroden (6, 8) bewirkte Spannung bzw. Strom mittels einer Auswerte-Einrichtung (21) überprüft, wobei bei Über- bzw. Unterschreiten eines vorgegebenen Grenzwertes ein Warnsignal (22) abgegeben wird.

0148312

Schaltungsanordnung zum Überprüfen der
Lage von Elektroden.

Die Erfindung betrifft eine Schaltungsanordnung zum Überprüfen der Lage von Elektroden auf einen Gegenstand, der insbesondere mit von einem Generator an die Elektroden gelieferter elektrischer Energie zu behandeln ist. Bei einer solchen Anordnung können die Elektroden aus einer exakten Position verrutschen; sie können z.B. sich berühren (Kurzschlußfall), abfallen (Öffnungsfall), oder einen zu großen oder zu kleinen Abstand voneinander oder gegenüber dem Gegenstand haben. Dadurch wird unter Umständen die Zufuhr der elektrischen Energie unerwünscht beeinflußt; im einfachsten Fall kann dadurch eine zu geringe Wirkung auftreten, es ist aber auch eine gefährliche Überlastung möglich. Wenn z.B. die Elektroden von einem Muskelstimulationsgerät genommen werden, können Hautverbrennungen auftreten.

Die Erfindung löst die Aufgabe, die Lage der Elektroden, insbesondere auch laufend während des Behandlungsvorganges, überprüfen zu können, wobei bei einer unzulässigen Abweichung der Elektrodenlage ein Signal abgegeben wird, damit selbsttätig oder durch das Bedienungspersonal eingegriffen werden kann.

Zur Lösung dieser Aufgabe wird eine gattungsgemäße Schaltungsanordnung vorgeschlagen, die erfindungsgemäß in der Weise ausgebildet ist, daß ein elektrischer Prüfimpuls periodisch an die Elektroden angelegt wird und die bzw. der durch den Prüfimpuls an bzw. in der Übergangsimpedanz zwischen den Elektroden bewirkte Spannung bzw. Strom mittels einer Auswerteeinrichtung überprüft wird und daß bei Über- bzw. Unterschreiten eines vorgegebenen Grenzwertes ein Warnsignal abgegeben wird.

Wenn die Übergangsimpedanz praktisch nur einen ohmschen Anteil besitzt, kann der während des Prüfimpulses auftretende Strom bzw. Spannungsabfall ausgewertet, der Übergangswiderstand bestimmt und mit einem ggf. je nach Behandlungsfall eingestellten Sollwert verglichen werden.

Eine weitere Lösung der Erfindung besteht darin, daß die Elektroden wenigstens annähernd gegeneinander isoliert sind und so die Übergangsimpedanz im Normalfall einen merklichen kapazitiven Anteil aufweist, daß die durch den Prüfimpuls bewirkten Ladungs- bzw. Spannungsänderungen am kapazitiven Anteil in der Auswerteeinrichtung bestimmt werden und daß bei Über- bzw. Unterschreiten eines vorgegebenen Grenzwertes ein Warnsignal abgegeben wird.

Die Bestimmung des ohmschen bzw. des kapazitiven Anteiles der Übergangsimpedanz mittels der Prüfimpulse kann grundsätzlich gleichzeitig mit der Zuführung der Behandlungsenergie erfolgen. Wenn z.B. Kurzwellenenergie mit einer Frequenz von etwa 27 MHz zugeführt wird und die Prüfimpulse eine Wiederholungsfrequenz von 50 Hz aufweisen, ist eine Trennung durch frequenzabhängige Filter möglich. Besonders einfach ist die Entkopplung zwischen den Prüfimpulsen und der Behandlungsenergie dann, wenn der Generator wenigstens zweitweise, z.B. durch Abschalten, eine hohe innere Impedanz aufweist und der Prüfimpuls in diesen Intervallen hoher Generator-Innenimpedanz angelegt wird.

Besonders einfach ist die Entkopllung, wenn der Prüfimpuls dann zugeführt wird, wenn die Behandlungsenergie nicht zugeführt wird, z.B. vor dem Einschalten oder in periodischen Pausen der Behandlungsenergie.

Eine bevorzugte Ausführungsform der Erfindung ist dadurch gekennzeichnet, daß der Prüfimpuls den Elektroden als Stromimpuls zugeführt wird und daß die während seiner Dauer an den Elektroden auftretende Spannung der Auswerte-Einrichtung zugeführt wird, die ein Abschaltsignal für den Generator liefert, wenn die Elektroden-Spannung einen oberen Grenzwert überschreitet oder einen unteren Grenzwert unterschreitet und/oder wenn die Kapazität des kapazitiven Anteils zwischen den Elektroden einen vorgegebenen maximalen Wert über- und/oder einen vorgegebenen minimalen Wert unterschreitet. Das Warnsignal wird dabei unmittelbar zum Abschalten des Generators benutzt, und es können sowohl für den ohmschen wie für den kapazitiven Anteil der Übergangsimpedanz nach oben und nach unten Grenzwerte festgelegt werden.

Zweckmäßig weist der Prüfimpuls abwechselnd positive und negative Polarität auf. Dadurch wird ein Fließen eines Gleichstromanteiles durch den Behandlungsgegenstand vermieden, der sonst zu elektrolytischen Zersetzungen oder anderen Schädigungen führen könnte.

Nach einer weiteren Ausführungsform kann die Kapazität durch Auswerten der Spannungsänderung während des Prüfimpulses oder unmittelbar nach seinem Abschalten bestimmt werden. Da durch den Prüfimpuls eine Aufladung bzw. Entladung der Kapazität erfolgt, ergibt sich während der Dauer des Prüfimpulses eine, bekanntlich etwa exponentiell verlaufende Spannungsänderung an der Übergangsimpedanz. Nach Wegfall des Prüfimpulses ist auf der Kapazität eine Ladungs-änderung erfolgt und es läßt sich eine entsprechende Spannungsänderung feststellen, die entsprechend der gegebenen Zeitkonstante ebenfalls etwa exponentiell wieder zurückgeht. Es ist unmöglich, während des Prüfimpulses oder nach seinem Wegfall durch mehrere Meßpunkte den Verlauf der Spannungskurve festzustellen und daraus die Kapazität zu bestimmen. Dabei enthält während des Prüfimpulses die Spannung auch den durch den ohmschen Widerstand bedingten Anteil, während nach dem Abschalten des Prüfimpulses praktisch allein die Spannung an der Kapazität festgestellt werden kann. Nach einer vereinfachten Ausführungsform kann daher die Kapazität durch Auswerten der Spannung unmittelbar nach dem Abschalten des Prüfimpulses bestimmt werden, wobei eine Berücksichtigung des exponentiellen Spannungsverlaufes nicht erforderlich ist.

Schließlich kann nach einer weiteren Ausführungsform das Warnsignal, z.B. in seiner Amplitude, von der Größe der festgestellten ohmschen und/oder kapazitiven Komponente abhängig sein und einer Einrichtung zur Einstellung der Elektroden-Lage, z.B. des Abstandes von dem Gegenstand oder des Auflagedruckes auf den Gegenstand oder einer Einrichtung zum Einstellen der vom Generator gelieferten elektrischen Energie regelnd zugeführt werden. So können, jedenfalls in einem bestimmten Bereich von Abweichungen, etwa erforderliche Korrekturen ohne Einschaltung des Bedienungs personals selbsttätig korrigiert werden.

Im folgenden wird der Gegenstand der Erfindung in den Zeichnungen erläutert. Es zeigt

Fig. 1    eine Schaltungsanordnung nach der Erfindung,

Fig. 2    den Prüfimpuls und (gestrichelt) den an der Übergangsimpedanz zwischen den Elektroden auftretenden Spannungsverlauf und

Fig. 3    ein gegenüber Fig. 1 in der Auswerte-Einrichtung und in dieser zugeordneten Stufen mehr detailliertes Schaltbild.

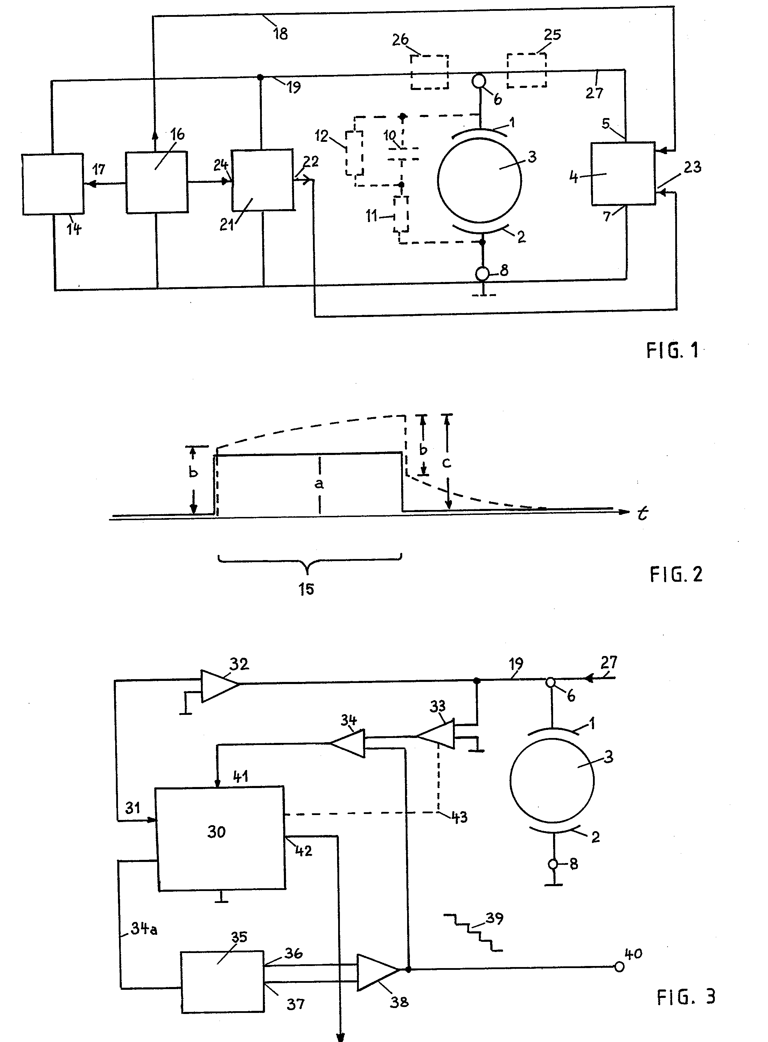

In Fig. 1 ist eine Schaltungsanordnung nach der Erfindung in einem Muskelstimulationsgerät dargestellt. Eine erste Elektrode 1 und eine zweite Elektrode 2 sind an einem Behandlungsgegenstand 3, z.B. einem durch seine Umfangslinie im Querschnitt dargestellten Oberarmmuskel, angeordnet. Ein Behandlungsgenerator 4 liefert von seinem ersten Ausgang 5 über eine erste Klemme 6 an die Elektrode 1 und von seinem zweiten Ausgang 7 über eine zweite Klemme 8 an die Elektrode 2 elektrische Energie. Diese kann aus Impulsen bestehen, deren Breite zwischen 10 µs und 990 µs einstellbar ist und deren Amplitude mit abwechselnder Polarität 160 $V_{ss}$ betragen kann, wobei während des Impulses ein konstanter Strom abgegeben wird, der zwischen 1 und 99 mA eingestellt werden kann. Die Frequenz dieser Impulse kann zwischen 1 und 99 Hz ebenfalls gewählt werden. Zwischen den Impulsen liegt somit ein Zwischenraum, in dem der Generator 4 abgeschaltet ist, keine Energie liefert und einen gegenüber den übrigen Schaltungsteilen hohen Innenwiderstand aufweist, so daß in den Pausen der Generatorzweig nicht weiter zu berücksichtigen ist.

Die Strecke zwischen den Elektroden 1 und 2 kann gemäß dem daneben gestrichelt dargestellten Ersatzschaltbild als Reihenschaltung eines Kondensators 10 von 0,47 µF und eines Widerstandes 11 von 1500 Ohm dargestellt werden, wobei dem Kondensator 10 ein Widerstand 12 von 220 Ohm parallel liegt. Dies sind natürlich ganz spezielle Werte eines Ausführungsbeispiels; in der Praxis können diese Werte im Einzelfall nach oben oder nach unten ganz erheblich abweichen.

Von einem Prüfsignalgenerator 14 werden den Klemmen 6 und 8 und damit an die Elektroden 1 und 2 Prüfimpulse zugeführt, die einer Spannungsquelle von 5 Volt mit 15 kOhm Innenwiderstand entsprechen. Bei den gegebenen Werten wird somit vom Prüfsignal-Generator 14 der Übergangsimpedanz 10, 11, 12 ein nahezu konstanter Strom zugeführt, wobei der Prüfimpuls über eine Dauer von 220 µs in den Pausen der Behandlungsimpulse, in denen der Generator 4 praktisch abgeschaltet ist, zugeführt werden.

Ein solcher Prüfimpuls ist schematisch mit der ausgezogenen Linie und einer Stromamplitude a dargestellt. Insbesondere infolge des Widerstandes 11 ergibt sich dadurch am Anfang des Impulsintervalles eine Spannungsamplitude b. Da jedoch im Laufe des Impulses die Kapazität 10 aufgeladen wird, nimmt die zwischen den Punkten 6 und 8 auftretende Spannung zu und erreicht am Ende des Impulsintervalles den etwas höheren Wert c, wobei also die Differenz zwischen dem Wert c und dem Wert b durch die Aufladung der Kapazität bedingt ist.

Wenn der Prüfimpuls (a) wegfällt, geht die in Fig. 2 gestrichelt dargestellte Spannung zwischen den Klemmen 6 und 8 momentan um den Betrag von b zurück, weil ja der Strom durch den Widerstand 11 momentan wegfällt. Es bleibt dann noch die Spannung (c - b) vom Kondensator 10 stehen, die dann exponentiell, etwa entsprechend der durch den Kondensator 10 und den Widerstand 12 bedingten Zeitkonstante, abklingt. Aus dem exponentiellen Verlauf der gestrichelten Kurve während des Prüfimpulses 15 oder danach kann, z.B. durch Amplitudenmessung an mehreren Punkten, die Größe der Kapazität bestimmt werden. Insbesondere für die beabsichtigte Überwachung und Warnung kann es ausreichend sein, allein die nach Abschaltung des Prüfimpulses 15 stehenbleibende Restspannung zum Vergleich mit einem Sollwert und ggf. zur Bildung des Warnsignales auszuwerten.

Von einer Steuerstufe 16 werden über eine Leitung 17 an den Prüfimpulsgenerator 14 und über eine Leitung 18 an den Behandlungs-Generator 4 Steuersignale gegeben, durch die sichergestellt wird, daß die Prüfimpulse in den Pausen der Behandlungsimpulse auftreten. Die während der Prüfimpulse 15 an der Klemme 6 gegenüber der (Masse. bzs. Erd-) Klemme 8 auftretende Spannung, die in Fig. 2 gestrichelt dargestellt ist, wird von der Verbindungsleitung 19 zwischen der Klemme 6 und dem Prüfimpuls-Generator 14 einem Eingang 20 einer Auswerteeinrichtung 21 zugeführt. In dieser werden die Amplitudenwerte (b, c) der in Fig. 2 gestrichelten Spannungskurve gemessen und mit mindestens einem, vorzugsweise einstellbaren, Sollwert verglichen. Diese Einstellung kann je nach dem zu behandelnden Gegenstand vorgenommen werden, um eine optimale Energiezufuhr sicherzustellen. Wenn eine unzulässige Abweichung von wenigstens einem Sollwert festgestellt wird, tritt am Ausgang 22 der Auswerte-Einrichtung 21 ein Warnsignal auf, das dem Behandlungsgenerator 4 am Eingang 23 zugeführt wird und diesen Generator abschaltet sowie ggf. ein Signal, z.B. eine blinkende Lampe oder eine Klingel, auslöst. Die Steuerstufe 16 kann auch die erforderlichen Tastsignale an einen Eingang 24 liefern, damit in der Auswerte-Einrichtung 21 die Amplitudenprüfung der Spannung zwischen den Klemmen 6 und 8 zu den richtigen Zeitpunkten erfolgt, die für eine zutreffende Auswertung zu wählen sind.

Die Prüfimpulse haben zweckmäßig alternierende Polarität, damit durch sie kein Gleichstrom durch den Behandlungsgegenstand hervorgerufen wird.

Insbesondere, wenn die Behandlungsenergie im Frequenzbereich sehr hoch liegt und Prüfimpulse niedriger Frequenz verwendet werden, kann es zulässig sein, daß die Behandlungsenergie durchlaufend wirksam ist. Dann sollten entsprechende Trennfilter 25 und 26 in die Leitung 27 zwischen der Klemme 6 und den Behandlungsgenerator 4 bzw. in die Leitung 19 zwischen der Klemme 6 und der Auswerte-Einrichtung 21 bzw. dem Prüfsignalgenerator 14 eingeschaltet werden.

In Fig. 3 sind wieder der Gegenstand 3 dargestellt zwischen den Elektroden 1 und 2, die an die Klemme 6 mit den Leitungen 19 und 27 und an die Klemme 8 angeschlossen sind. Der Behandlungsgenerator 4 ist weggelassen. Die Schaltung nach Fig. 3 wird gesteuert von einer Zentraleinheit 30, die insbesondere einen Tastgenerator enthält und die erforderlichen Steuer- und Prüf-Signale liefert, sowie die Auswertung der an der Klemme 6 gegenüber der Klemme 8 auftretenden Spannung vornimmt.

Vom Ausgang 31 der Zentraleinheit 30 werden die Prüfimpulse einem Verstärker 32 zugeführt, an dessen Ausgang die Leitung 19 angeschlossen ist. Die an der Klemme 6 auftretende Spannung wird über einen Verstärker 33 dem Eingang eines Vergleichs verstärkers 34 zugeführt. Die Zentraleinheit 30 gibt über eine Leitung 34a Steuersignale ab für einen Treppengenerator 35, an dessen Ausgänge 36 und 37 die Eingänge eines Differenzverstärkers 38 angeschlossen sind. Die Steuerung des Verstärkers 38 erfolgt derart, daß an seinem Ausgang während des Prüfimpulses 15 eine treppenförmig verlaufende Spannung auftritt, die in Fig. 3 bei 39 angedeutet ist.

Diese Treppenspannung 39 wird einerseits einer Klemme 40 zugeführt und kann von dort für andere Funktionen in dem betreffenden Gerät ausgenutzt werden. Die Treppenspannung 39 wird außerdem dem zweiten Eingang des Vergleichsverstärkers 34 zugeführt, der z.B. so eingestellt wird, daß er ein Ausgangssignal dann liefert, wenn die vom Ausgang des Verstärkers 33 erhaltene und dem ersten Eingang des Verstärkers 34 zugeführte Meßspannung größer ist als die dem zweiten Eingang zugeführte Treppenspannung 39. Entsprechend der zeitlichen Lage erhält man somit am Ausgang des Vergleichsverstärkers 34 ein Signal, das für die betreffende Amplitudenstufe repräsentant ist. Dieses Signal wird dem Eingang 41 der Auswertestufe 30 zugeführt. Das Signal 39 tritt vorzugsweise im Anfangsbereich des Prüfimpulses 15 und außerdem unmittelbar nach dessen Ende auf. Somit werden am Eingang 41 zwei Werte zugeführt, von denen der erste im wesentlichen dem Amplitudensprung b in Fig. 2 entspricht, während der zweite im wesentlichen dem Amplitudensprung (c - b) in Fig. 2 unmittelbar nach Beendigung des Prüfimpulses 15 entspricht. Der erste Amplitudensprung macht eine Aussage über die Größe des Widerstandes 11, und von der Zentraleinheit 30 wird am Ausgang 42 ein Warnsignal abgegeben, wenn dieser Widerstandswert oberhalb oder unterhalb eines eingestellten Sollbereiches liegt.

Der zweite Amplitudensprung repräsentiert die Größe des Kondensators 10. Auch dieser Amplitudensprung wird in entsprechender Weise gegenüber vorgegebenen Sollwerten ausgewertet, und wenn eine dieser Grenzen überschritten wird, wird ein entsprechendes Warnsignal über den Ausgang 42 abgegeben.

- 9 -  0148312

Das Warnsignal kann in entsprechender Weise wie bei Fig. 1 zum Ausschalten des Behandlungsgenerator 4 und zum Einschalten einer Warnlampe oder einer Warn-Klingel ausgenutzt werden.

Da die Amplitude (c - b) der durch die Kapazität 10 bedingten exponentiellen Änderung der Spannung zwischen den Klemmen 6 und 8 beträchtlich kleiner sein kann als der durch die Ohm'sche Komponente 11 hervorgerufene Spannungssprung am Anfang bzw. am Ende des Prüfimpulses, kann es zweckmäßig sein, den Verstärkungsgrad des Verstärkers 33 zum Bestimmen des exponentiellen Abfalles nach Ende des Prüfimpulses auf einen höheren, z.B. dreifachen, Wert umzuschalten. Dies kann von der Zentraleinheit 30 aus über die Verbindung 43 zum Verstärker 33 erfolgen.

Patentansprüche

1. Schaltungsanordnung zum Überprüfen der Lage von Elektroden auf einen Gegenstand, der insbesondere mit von einem Generator an die Elektroden gelieferter elektrischer Energie zu behandeln ist, dadurch gekennzeichnet, daß ein elektrischer Prüfimpuls (15) periodisch an die Elektroden (6, 8) angelegt wird und die bzw. der durch den Prüfimpuls (15) an bzw. in der Übergangsimpedanz (10,11,12) zwischen den Elektroden (6,8) bewirkte Spannung bzw. Strom mittels einer Auswerte-Einrichtung (21) überprüft wird und daß bei Über- bzw. Unterschreiten eines vorgegebenen Grenzwertes ein Warnsignal (22) abgegeben wird.

2. Schaltungsanordnung nach Anspruch 1, dadurch gekennzeichnet, daß die Elektroden (6,8) wenigstens annähernd gegeneinander isoliert sind und so die Übergangsimpedanz (10,11,12) im Normalfall einen merklichen kapazitiven Anteil (10) aufweist, daß die durch den Prüfimpuls (15) bewirkten Ladungs- bzw. Spannungsänderungen am kapazitiven Anteil (10) in der Auswerte-Einrichtung (21) bestimmt werden und daß bei Über- bzw. Unterschreiten eines vorgegebenen Grenzwertes ein Warnsignal (22) abgegeben wird.

3. Schaltungsanordnung nach Anspruch 1 oder 2, dadurch gekennzeichnet, daß bei einem Generator (4), der wenigstens zeitweise, z.B. durch periodisches Abschalten, eine hohe innere Impedanz aufweist, der Prüfimpuls (15) in diesen Intervallen hoher Generator-Innenimpedanz angelegt wird.

4. Schaltungsanordnung nach einem der vorangehenden Ansprüche, dadurch gekennzeichnet, daß der Prüfimpuls (15) zugeführt wird, wenn die Behandlungsenergie (von 4) nicht zugeführt wird, z.B. vor dem Einschalten oder in periodischen Pausen des Generators (4).

5. Schaltungsanordnung nach einem der vorangehenden Ansprüche, dadurch gekennzeichnet, daß das Warnsignal (21) einer Einrichtung zum Abschalten des Generators (4, 23) zugeführt wird.

6. Schaltungsanordnung nach einem der vorangehenden Ansprüche, dadurch gekennzeichnet, daß der Prüfimpuls (15) den Elektroden (6,8) als Stromimpuls zugeführt wird und daß die während seiner Dauer an den Elektroden (6,8) auftretende Spannung der Auswerte-Einrichtung (21) zugeführt wird, die ein Abschaltsignal (22,23) für den Generator (4) liefert, wenn die Elektroden-Spannung einen oberen Grenzwert überschreitet oder einen unteren Grenzwert unterschreitet und/oder wenn die Kapazität (10) des kapazitiven Anteils der Übergangsimpedanz zwischen den Elektroden (6,8) einen vorgegebenen maximalen Wert über- oder einen vorgegebenen minimalen Wert unterschreitet.

7. Schaltungsanordnung nach einem der vorangehenden Ansprüche, dadurch gekennzeichnet, daß der Prüfimpuls (15) abwechselnd positive und negative Polarität aufweist.

8. Schaltungsanordnung nach einem der vorangehenden Ansprüche, dadurch gekennzeichnet, daß die Kapazität (10) der Übergangsimpedanz durch Auswerten der Spannungsänderung während des Prüfimpulses (15) oder unmittelbar nach seinem Abschalten bestimmt wird.

9. Schaltungsanordnung nach einem der vorangehenden Ansprüche, dadurch gekennzeichnet, daß die Kapazität (10) durch Auswerten der Spannung unmittelbar nach dem Abschalten des Prüfimpulses (15) bestimmt wird.

10. Schaltungsanordnung nach einem der vorangehenden Ansprüche, dadurch gekennzeichnet, daß das Warnsignal (22), z.B. in seiner Amplitude, von der Größe der festgestellten ohmschen und/oder kapazitiven Komponente (11 bzw.

- 12 -  0148312

10) abhängig ist und einer Einrichtung zur Einstellung der Elektrodenlage, z.B. des Abstandes von dem Gegenstand (3) oder des Auflagedruckes auf den Gegenstand (3) regelnd zugeführt wird.

0148312

1/1

FIG. 1

FIG. 2

FIG. 3